# FASCICULE DE BREVET EUROPEEN

(11) **EP 1 084 138 B1**
(45) Date de publication et mention de la délivrance du brevet: **30.01.2002**
(21) Numéro de dépôt: 99923656.5
(22) Date de dépôt: 03.06.1999
(51) Int. Cl.: C07K 5/075

(54) **PROCEDE DE PREPARATION DE L'ASPARTYL CYCLOHEXYLALANINAMIDE**
VERFAHREN ZUR HERSTELLUNG VON ASPARTYL-CYCLOHEXYLALANINAMID
METHOD FOR PREPARING ASPARTYL CYCLOHEXYLALANINAMIDE

(30) Priorité: 05.06.1998 FR 9807069; 15.10.1998 US 104417 P
(43) Date de publication de la demande: 21.03.2001
(73) Titulaire: Aventis Pharma S.A., 92160 Antony (FR)
(72) Inventeur: STAMMLER, Robert, F-75012 Paris (FR); DAUBIE, Christophe, F-75005 Paris (FR); LAVIGNE, Michel, F-91380 Chilly Mazarin (FR)
(86) Numéro de dépôt international: FR9901300
(87) Numéro de publication internationale: WO9964443

(56) Documents cités:
- EP-A- 0 405 506
- WO-A-98/46629

## Description

La présente invention concerne un procédé de préparation amélioré de l'α-aspartyl-β-cyclohexylalaninamide à partir d'un ester de l'α-aspartyl phénylalanine.

L'α-aspartyl phénylalaninamide est habituellement préparée par couplage peptidique de dérivés d'acide aspartique et de phénylalaninamide. Ces couplages nécessitent des protections de l'acide aspartique et le phénylalaninamide doit être préparé à partir de phénylalanine. Après couplage peptidique, les déprotections d'amine et d'acide sont nécessaires pour accéder à l'α-aspartyl phénylalaninamide, rendant ainsi sa synthèse longue, peu performante et coûteuse [J. Org. Chem., 40, 2495 (1975) ; WO 9006937 ; DD 209191 ; DE 2245459 ; EP149582].

Dans la demande de brevet WO 95/10295 a été décrite la préparation de dérivés pseudotétrapeptides de formule générale : 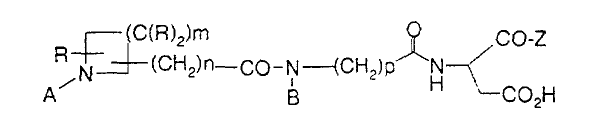 dans laquelle notamment Z représente : 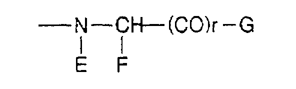 pour lequel E est notamment hydrogène, F est notamment cycloalcoylalcoyle, r peut être 1 et G peut former un groupe NR₁R₂.
Ces dérivés, actifs dans le domaine cardiovasculaire, se préparent par l'intermédiaire de dérivés aspartiques tels que par exemple le dérivé aspartique de formule générale : 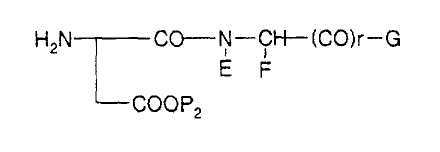 cependant le procédé est assez long et coûteux, puisqu'il fait intervenir des matières de départ comme la cyclohexylalanine et le monobenzylester de l'acide benzyloxycarbonyl aspartique, non commerciales, surtout à l'échelle industrielle, matières premières qui doivent être préalablement protégées avant leur mise en oeuvre dans le procédé. Ainsi la préparation industrielle du produit biologiquement actif est extrêmement laborieuse.

Dans la demande de brevet EP 405506 a été décrite la préparation de l'α-aspartyl cyclohexylalaninamide par hydrogénation catalytique de l'α-aspartyl phénylalaninamide. Cependant l'α-aspartyl phénylalaninamide préparé par des méthodes classiques ne pouvait pas permettre d'obtenir une amélioration globale du procédé.

Il a maintenant été trouvé, et c'est ce qui fait l'objet de la présente invention, que l'on pouvait préparer le sel d'ammonium de l'α-aspartyl phénylalaninamide de formule : 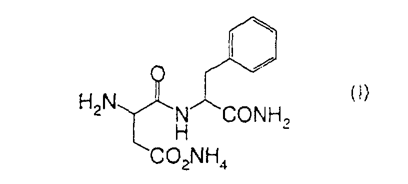 directement à partir d'un ester de l'α-aspartyl phénylalanine de formule générale : 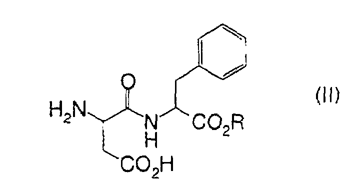 dans laquelle R est un radical alcoyle contenant 1 à 4 atomes de carbone et que l'on pouvait de ce fait préparer l'α-aspartyl-β-cyclohexylalaninamide ou son sel en seulement 2 étapes.

Selon l'invention, la préparation du sel d'ammonium de l'α-aspartyl phénylalaninamide est mise en oeuvre par amidification de l'ester de l'α-aspartyl phénylalanine de formule générale (II), puis dans une deuxième étape l'α-aspartyl-β-cyclohexylalaninamide est obtenu par hydrogénation catalytique du produit obtenu, éventuellement libéré préalablement de son sel (éventuellement in situ).

Selon un mode préféré de l'invention, le procédé est mis en oeuvre sur l'ester de la (L)-α-aspartyl-(L)-phénylalanine pour préparer le (L)-α-aspartyl-(L)-phénylalaninamide puis le (L)-α-aspartyl-(L)-β-cyclohexylalaninamide.

Egalement selon un mode préféré, l'invention peut être mise en oeuvre à partir de l'ester méthylique de l'α-aspartyl phénylalanine.

Selon l'invention la réaction d'amidification est mise en oeuvre par action de l'ammoniac liquide, à une température comprise entre -40 et 20°C. Eventuellement elle peut être mise en oeuvre en présence d'un cosolvant comme l'eau, un alcool (méthanol, éthanol, i.propanol, éthylèneglycol) ou l'acétonitrile.

Lorsque l'on souhaite libérer le produit de son sel pour obtenir intermédiairement le (L)-α-aspartyl-(L)-phénylalaninamide, le sel d'ammonium obtenu précédemment peut être soit directement chauffé pendant 12 à 16 heures à une température comprise entre 30 et 40°C, sous balayage d'azote et éventuellement sous pression réduite, soit traité en milieu acide aqueux comme décrit ci-après dans les exemples. Notamment par des acides tels que décrits ci-après.

Lorsque l'on souhaite libérer le produit de son sel et obtenir un sel d'acide, le sel d'ammonium est traité en milieu acide selon les méthodes habituelles qui n'altèrent pas le reste de la molécule et comme décrit ci-après dans les exemples. Notamment, et à titre non limitatif, par des acides tels que l'acide chlorhydrique, l'acide tartrique, l'acide acétique, l'acide oxalique, l'acide lactique, l'acide citrique, l'acide mandélique.

L'étape d'hydrogénation catalytique est mise en oeuvre à une température comprise entre 20 et 60°C (de préférence à 40°C), sous une pression d'hydrogène de 1 à 8 bars (de préférence 4 bars), en milieu aqueux chlorhydrique ou en milieu acétique, en présence de platine, et éventuellement en présence d'un autre acide organique comme par exemple l'acide trifluoracétique, l'acide trichloroacétique, l'acide oxalique, malonique, l'acide citrique, l'acide tartrique, l'acide malique, l'acide formique, l'acide lactique.

Les dérivés obtenus par le procédé selon l'invention peuvent éventuellement être transformés en sels d'addition avec les acides.

Parmi les sels industriellement intéressants peuvent être cités le chlorhydrate, bromhydrate, tartrate, acétate, oxalate, lactate, citrate, mandélate, trifluoracétate.

Le procédé selon l'invention est particulièrement intéressant du fait qu'il ouvre la route à la préparation de l'α-aspartyl-(3-cyclohexylalaninamide en 2 étapes seulement et aussi du fait qu'il permet de préparer l'amide intermédiaire de formule (I) en une seule étape à partir d'une matière première facilement accessible industriellement : l'aspartame.

L'α-aspartyl-β-cyclohexylalaninamide ainsi obtenu peut être purifié selon les méthodes habituelles telles que la chromatographie ou la cristallisation.

L'α-aspartyl-β-cyclohexylalaninamide peut être mis en oeuvre pour la préparation de dérivés pseudotetrapeptides en opérant selon la méthode décrite dans la demande internationale WO 95/10295.

Les exemples suivants donnés à titre non limitatif illustrent la présente invention.

### Exemple 1

Dans un réacteur double-enveloppe de 2 litres refroidi par circulation à -40°C, on charge 500 cm³ d'ammoniac liquide puis on ajoute en 30 minutes sous agitation 250 g d'ester méthylique de la (L)-α-aspartyl-(L)-phénylalanine. On obtient une solution incolore, agitée pendant encore 1 heure à -40°C. La circulation en double enveloppe est arrêtée et on laisse le mélange se réchauffer et dégazer sous agitation. En 2 heures la solution se concentre, épaissit et devient visqueuse, on arrête l'agitation et on laisse dégazer. Le mélange cristallise sous forme de meringue. Le dégazage est terminé sous pression réduite (4 heures sous 5,3 kPa). Après avoir purgé à l'azote, on obtient 239 g du sel d'ammonium du (L)-α-aspartyl-(L)-phénylalaninamide, avec un rendement de 95%.

Spectre RMN ¹H, DMSO-d6, T=300°K, δ en ppm (300 MHz) : 2,15 et 2,42 (1H chacun, respectivement dd: J=9 et 16Hz, et dd: J=5 et 16Hz, COCH₂), 2,86 et 3,07 (1H chacun, respectivement dd: J=9 et 14Hz, et dd: J=4 et 14Hz, PhCH₂), 3,54 (1H, q, J=4 et 9Hz, NCH), 4,40 (1H, m, NCH), 7,10 (1H, s, ½ CONH₂), 7,25 (5H, m, phényle), 7,74 (1H, s, ½ CONH₂), 8,60 (1H, s large, NH).

Spectre infra-rouge, comprimé KBr, cm⁻¹: large bande de 3600 à 2000, ν N-H liés dont ν NH₄⁺ avec maxima vers 3388 + 3284 + 3190, ν N-H des amides, 3086 + 3063 + 3029, ν C-H du benzène monosubstitué ; bandes vers 1673, ν C=O de l'amide primaire, 1637 principalement ν C=O de l'amide secondaire, 1567 principalement ν_{*as*} C=O de COO⁻ + δ NH de l'amide secondaire, 1496 + 1454 C=C noyau benzénique monosubstitué, 1444 déformation de CH₂, 1393 ν_{*s*} C=O de COO⁻ + δ NH₄⁺, 1031 noyau benzénique monosubstitué, 750 γ C-H du noyau benzénique monosubstitué, 698 déformation du noyau benzénique monosubstitué.

Dans un réacteur agité double-enveloppe de 2 litres refroidi par circulation à 10°C, on charge 500 cm3 d'eau et 250 cm³ d'acide chlorhydrique 6 N, on ajoute en 1 heure à 10-15°C, 239 g du sel d'ammonium du (L)-α-aspartyl-(L)-phénylalaninamide préparé précédemment, en solution dans 250 cm³ d'eau, puis on ajuste à pH = 1 avec de l'acide chlorhydrique 6N. La solution acide légèrement trouble est clarifiée par filtration sur célite, rincée par 300 cm³ d'eau. On obtient une solution limpide et incolore (1650 g).

La moitié de cette solution (825 g) est chargée dans un hydrogéneur de 4 litres, puis on ajoute 65 g de platine 5% sur alumine à 50% d'eau en suspension dans 1200 cm³ d'eau. L'hydrogéneur est fermé, purgé à l'azote puis à l'hydrogène. On chauffe la masse réactionnelle à 35°C sous agitation, puis on monte la pression d'hydrogène à 4 bars. L'hydrogénation est complète en 2 heures, la masse est refroidie à 20°C, l'appareil est purgé à l'azote et le mélange réactionnel est filtré sur verre fritté. La solution limpide et incolore est amenée à pH = 7,9 par addition de soude N, le mélange précipite. La suspension obtenue est filtrée sur verre fritté, le gâteau est lavé avec 3 fois 200 cm³ d'eau et séché à 40°C sous 1,33 kPa pendant 16 heures.

On obtient 102,3 g de (L)-α-aspartyl-(L)-β-cyclohexylalaninamide, avec un rendement de 89% vis à vis de l'ester méthylique de la (L)-a-aspartyl-(L)-phénylalanine engagé initialement.

Spectre de RMN ¹H dans DMSO-d6, T=300°K, δ en ppm (300 MHz) : entre 0,70 et 1,80 (13H, m, 6 CH₂ et 1 CH) ; 2,28 et 2,52 (1H chacun, respectivement dd, J=9 et 16Hz, et dd, J=5 et 16Hz, CH₂CO) ; 3,75 (1H, q, J=5 et 9Hz, NCH) ; 4,20 (1H, m, NCH) ; 6,95 et 7,60 (1H chacun, s, CONH₂) ; 8,65 (1H, d, J=5Hz, NH).

Spectre infra-rouge, comprimé KBr, cm⁻¹ : large bande de 3600 à 2200 ν N-H liés dont ν NH₃⁺ avec maximums vers 3469 + 3365 + 3264 + 3180 + 3063 ν N-H des amides et NH₃⁺, 2922 + 2851 ν C-H du cyclohexane. 2669 + 2605 sel d'amine primaire ; bandes vers 1671 ν C=O de l'amide primaire, 1636 principalement ν C=O de l'amide secondaire, 1596 principalement ν_{*as*} C=O de COO⁻ + déformation de NH₃⁺. 1555 δ NH de l'amide secondaire, 1445 déformation de CH₂, 1396 + 1384 principalement ν_{*s*} C=O de COO⁻.

### Exemple 2

Aux 239 g du sel d'ammonium du (L)-α-aspartyl-(L)-phénalalaninamide obtenus à l'exemple 1 sont ajoutés 956 cm³ d'eau. Le mélange est maintenu tel quel pendant 30 minutes puis agité jusqu'à dissolution complète. A cette solution maintenue sous agitation à 15-25°C est ajoutée en 60 minutes une solution aqueuse normale d'acide chlorhydrique jusqu'à pH=6,0. Le (L)-α-aspartyl-(L)-phénylalaninamide précipite et on obtient une bouillie épaisse qui est filtrée sur verre fritté, lavée par 239 cm³ d'eau. Le gâteau est essoré à fond puis séché en étuve à 40±5°C sous 1,33 kPa pendant 12 heures. On obtient 176 g de (L)-α-aspartyl-(L)-phénylalaninamide, avec un rendement de 78%.

Spectre de RMN ¹H dans le DMSO-d6, T=300°K, δ en ppm (250 MHz) : 2,3 et 2,5 (1H chacun, m, COCH₂) ; 2,9 et 3,1 (1H chacun, respectivement dd, J=16 et 6Hz, et dd, J=16 et 3Hz, CH₂Ph) ;, 3,8 (1H, m, NCH) ; 4,5 (1H, m, NCH) ; 7,2 (1H, s, ½ CONH₂) ; 7,3 (5H, m, phényl.) ; 7,8 (1H, s, ½ CONH2.) ; 8,90 (1H, s large, NH).

Spectre infra-rouge, comprimé KBr, cm⁻¹ : large bande de 3600 à 2000 ν N-H liés avec maximums vers : 3387 + 3318 + 3210 ν N-H des amides et 3031 + 2684 + 2628 ν NH₃⁺ ; bandes vers 1668 ν C=O de l'amide primaire, 1644 principalement ν C=O de l'amide secondaire, 1626 principalement δ N-H de l'amide primaire, 1550 principalement ν_{*as*} C=O de COO⁻ + δ NH de l'amide secondaire, 1496 + 1446 C=C noyau benzénique monosubstitué, 1394 ν_{*S*} C=O de COO- + δ, NH₄⁺ + C-CONH₂, 1032 noyau benzénique monosubstitué, 748 γ C-H du noyau benzénique monosubstitué, 704 cm⁻¹ déformation du noyau benzénique monosubstitué ; large bande de 800 à 400 avec maximum vers 634, ω N-H.

### Exemple 3

Aux 239 g du sel d'ammonium du (L)-α-aspartyl-(L)-phénylalaninamide obtenus à l'exemple 1 sont ajoutés 2390 cm³ d'eau. Le mélange est maintenu tel quel pendant 30 minutes puis agité jusqu'à dissolution complète. A cette solution maintenue sous agitation à 15-25°C sont ajoutés 60 g d'acide (L) tartrique. Le mélange est maintenu sous agitation pendant 3 heures, filtré sur verre fritté. Le gâteau est lavé par 239 cm³ d'eau puis séché en étuve à 40±5°C sous 1,33 kPa pendant 12 heures. On obtient 205 g de l'hémitartrate du (L)-α-aspartyl-(L)-phénylalaniamide, avec un rendement de 72%.

Spectre de RMN ¹H dans le DMSO-d6, T=300°K, δ en ppm (250 MHz) : 2,4 et 2,6 (1H chacun, respectivement dd, J=16 et 8Hz, et dd, J=16 et 3Hz, COCH₂) ; 2,9 et 3,1 (1H chacun, respectivement dd, J=16 et 10Hz, et dd, J=16 et 3Hz, CH₂Ph) ; 3,8 (1H, m, NCH) ; 4,0 (2H, s, ½ acide tartrique) ; 4,5 (1H, m, NCH) ; 7,2 (1H, s, ½ CONH₂) ; 7,3 (5H, m, phényl.) ; 7,7 (1H, s, ½ CONH2) ; 8,70 (1H, d, J=5Hz, NH).

Spectre infra-rouge, comprimé KBr, cm⁻¹ : large bande de 3700 à 2200 ν N-H liés dont ν NH₃⁺ et O-H avec maximums vers 3530 + 3495 + 3441+ 3390 + 3306 + 3190, ν O-H du tartrate + ν N-H des amides et NH₃⁺ + ν O-H de l'acide, 2502 principalement O-H acide (et sel d'amine primaire) ; bandes vers 1728 (épaulement) ν C=O de l'acide, 1668 ν C=O de l'amide primaire et ν C=O de l'amide secondaire, 1602 principalement νₐₛ C=O de COO⁻ + déformations NH de l'amide primaire et de NH₃⁺, 1532 δ NH de l'amide secondaire, 1497 + 1455 ν C=C noyau benzénique monosubstitué, 1474 déformation des OH du tartrate, 1395 νₛ C=O de COO⁻ + ν C-CONH2 de l'amide, 1304 ν C-O de l'acide, 1133 + 1081 ν C-OH du tartrate, 756 γ C-H du noyau benzénique monosubstitué, 703 déformation du noyau benzénique monosubstitué, 513 ω C-O des COO⁻ du tatrate ; large bande de 750 à 400 avec maximum vers 616 ω N-H.

### Exemple 4

Dans un ballon tricol de 1 litre agité, on charge 510 cm³ d'acide acétique, puis on ajoute 102,3 g de (L)-α-aspartyl-(L)-cyclohexylalaninamide. Après 30 minutes d'agitation, on obtient une solution limpide. On verse en 15 minutes sur cette solution, 30 cm³ d'acide chlorhydrique 12 N, le chlorhydrate précipite et on obtient une bouillie épaisse qui est filtrée sur verre fritté, lavée par 2 fois 100 cm³ d'acide acétique. Le gâteau est essoré à fond puis séché en étuve à 50°C sous 1,33 kPa pendant 16 heures.

On obtient 113 g du chlorhydrate du (L)-α-aspartyl-(L)-β-cyclohexylalaninamide avec un rendement global de 83% vis à vis de l'ester méthylique de la (L)-α-aspartyl-(L)-phénylalanine engagé initialement à l'exemple 1.

Spectre de RMN ¹H dans DMSO-d6, T=300°K. δ en ppm (300 MHz) : entre 0,7 et 1,8 (13H, m, CH₂C₆H₁₁) ; 2,8 et 3,0 (1H chacun, respectivement dd, J=8 et 16Hz, et dd, J=4 et 16Hz, CH₂CO) ; 4,1 (1H, m, CH) ; 4,3 (1H, m, CH) ; 7,1 et 7,4 (1H chacun, s, CONH₂) ; 8,7 (1H, d, J=8Hz, NH).

Spectre infra-rouge, comprimé KBr, cm⁻¹ : large bande de 3700 à 2200 cm⁻¹ ν N-H liés dont ν NH₃⁺ et O-H avec maximums vers 3432 + 3395 + 3362 + 3181 + 3046 ν N-H des amides et NH₃⁺ + ν O-H de l'acide, 2924 + 2852 ν C-H du cyclohexane, 2698 + 2636 principalement O-H acide (et sel d'amine primaire) ; bandes vers 1736 + 1715 ν C=O de l'acide, 1682 ν C=O de l'amide primaire, 1670 principalement ν C=O de l'amide secondaire, 1605 + 1586 principalement déformations NH de l'amide primaire et de NH₃⁺, 1556 δ NH de l'amide secondaire, 1450 déformation de CH₂, 1407 ν C-N de l'amine + déformation OH de l'acide, 1299 ν C-O de l'acide, 1201 non attribuée.

### Exemple 5

Le sel d'ammonium du (L)-α-aspartyl-(L)-phénylalaninamide est préparé comme décrit à l'exemple 1, puis mis en oeuvre directement dans les conditions suivantes :

Dans un hydrogéneur / cavitateur de 4 litres, on charge 1440 cm³ d'acide acétique, puis on ajoute 239 g du sel d'ammonium du (L)-α-aspartyl-(L)-phénylalaninamide en solution dans 240 cm³ d'eau. Sur la solution obtenue on verse 67 cm³ d'acide chlorhydrique 12 N en 15 minutes, puis on ajoute 130 g de platine 5% sur alumine à 50% d'eau. L'hydrogéneur est fermé, purgé à l'azote puis à l'hydrogène. On chauffe la masse réactionnelle à 55°C sous agitation, puis on monte la pression d'hydrogène à 4 bars. L'hydrogénation est complète en 3 heures, la masse est refroidie à 20°C, l'appareil est purgé à l'azote et le mélange réactionnel est filtré sur verre fritté. Le filtrat est chargé dans un ballon tricol de 4 litres muni d'une agitation, le chlorhydrate est précipité par addition de 67 cm³ d'acide chlorhydrique 12 N en 20 minutes. La bouillie est filtrée sur verre fritté et lavée par 2 fois 200 cm³ d'acide acétique. Le gâteau est essoré à fond puis séché en étuve à 50°C sous 1,33 kPa pendant 16 heures.

On obtient 114 g du chlorhydrate du (L)-α-aspartyl-(L)-β-cyclohexylalaninamide avec un rendement global de 84% vis à vis de l'ester méthylique de la (L)-α-aspartyl-(L)-phénylalanine engagé initialement et dont les caractéristiques physiques sont identiques à celles décrites précédemment à l'exemple 4.

### Exemple 6

Dans un réacteur double-enveloppe de 2 litres refroidi par circulation à -40°C, on charge 150 cm³ d'ammoniac liquide puis on ajoute en 15 minutes sous agitation 100 g de l'ester méthylique de la (L)-α-aspartyl-(L)-phénylalanine. On obtient une solution incolore qui est agitée pendant encore 10 heures à -40°C, puis on ajoute 900 cm³ de propanol-2 pendant une durée d'une heure en laissant la température remonter à 20°C. Le mélange cristallise. Il est encore maintenu sous agitation pendant 2 heures à 20°C, puis filtré sur verre fritté, lavé deux fois par 200 cm³ de propanol-2. On obtient 211 g du sel d'ammonium du (L)-α-aspartyl-(L)-phénylalaninamide, humide (contenant 54 % poids/poids de propanol-2) qui séchés à l'étuve à 35±5°C sous 1,33 kPa et sous balayage d'azote pendant 16 heures conduisent à 92,4 g de (L)-α-aspartyl-(L)-phénylalaninamide, avec un rendement de 97% et dont les caractéristiques physiques sont identiques à celle du produit décrit à l'exemple 2.

### Exemple 7

Dans un hydrogéneur de 2 litres, on charge 390 cm³ d'acide acétique, puis on ajoute 65 g de (L)-α-aspartyl-(L)-phénylalaninamide préparé comme décrit à l'exemple 6. Sur la solution obtenue, on verse 36 cm³ d'acide trifluoroacétique en 5 minutes, puis on ajoute 27 g de platine à 5% sur alumine à 50% d'eau. L'hydrogéneur est fermé, purgé à l'azote puis à l'hydrogène. On chauffe la masse réactionnelle à 35°C sous agitation, puis on monte la pression d'hydrogène à 4 bars. L'hydrogénation est complète en 1 heure, la masse est refroidie à 20°C, l'appareil est purgé à l'azote et le mélange réactionnel est filtré sur verre fritté et rinçé par 65 cm³ d'acide acétique. Le filtrat et le rinçage sont chargés dans un ballon tricol de 1 litre muni d'une agitation, le chlorhydrate est précipité par addition de 153 cm³ d'une solution acétique d'acide chlorhydrique 1,5 N en 15 minutes. La suspension est maintenue sous agitation à 20°C pendant 2 heures, filtrée sur verre fritté et lavée 2 fois par 65 cm³ d'acide acétique. Le gâteau est essoré à fond puis séché en étuve à 50°C sous 1,33 kPa pendant 16 heures.

On obtient 61 g du chlorhydrate du (L)-α-aspartyl-(L)-β-cyclohexylalaninamide avec un rendement de 81% et dont les caractéristiques physiques sont identiques à celle du produit décrit à l'exemple 5.

## Revendications

1. Un procédé de préparation de l'a-aspartyl-β-cyclohexylalaninamide, **caractérisé en ce que** l'on effectue dans une première étape l'amidification d'un ester de l'aspartyl phénylalanine de formule générale : 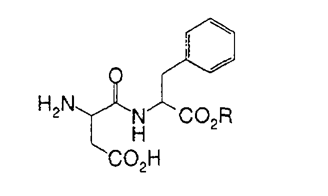 dans laquelle R est un radical alcoyle contenant 1 à 4 atomes de carbone, pour donner le sel d'ammonium de l'α-aspartyl phénylalaninamide de formule : 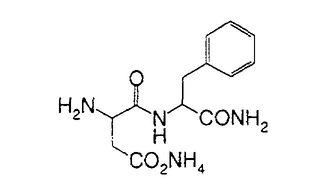 puis effectue l'hydrogénation catalytique du produit obtenu éventuellement libéré préalablement de son sel, et transforme éventuellement l'α-aspartyl-β-cyclohexylalaninamide obtenu en un sel d'addition avec un acide.

2. Procédé selon la revendication 1, **caractérisé en ce que** l'on prépare le (L)-α-aspartyl-(L)-β-cyclohexylalaninamide ou son sel d'addition avec un acide.

3. Un procédé de préparation de l'α-aspartyl phénylalaninamide de son sel d'ammonium ou de son sel d'addition avec un acide, **caractérisé en ce que** l'on effectue l'amidification d'un ester de l'aspartyl phénylalanine de formule générale : 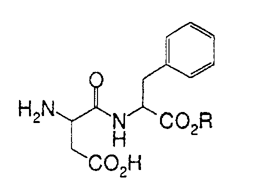 dans laquelle R est un radical alcoyle contenant 1 à 4 atomes de carbone, pour donner le sel d'ammonium de l'α-aspartyl phénylalaninamide de formule: 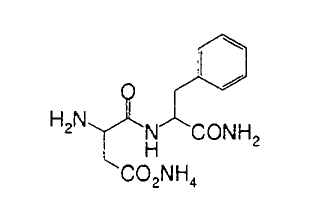 puis libère éventuellement le produit de son sel et transforme éventuellement en un sel d'addition avec un acide.

4. Procédé selon la revendication 3, **caractérisé en ce que** l'on prépare le (L)-α-aspartyl-(L)-phénylalaninamide, son sel d'ammonium ou son sel d'addition avec un acide.

5. Procédé selon la revendication 1 ou 3, **caractérisé en ce que** l'on met en oeuvre l'ester méthylique de l'α-aspartyl phénylalanine.

6. Procédé selon la revendication 1 ou 3, **caractérisé en ce que** la réaction d'amidification est mise en oeuvre par action de l'ammoniac liquide.

## Claims

1. A method for preparing (α-aspartyl)-β-cyclohexylalaninamide, **characterized in that**, in a first stage, an ester of aspartylphenylalanine of general formula: 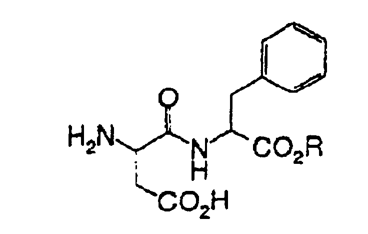 in which R is an alkyl radical comprising 1 to 4 carbon atoms, is amidated, to give the ammonium salt of (α-aspartyl)phenylalaninamide of formula: 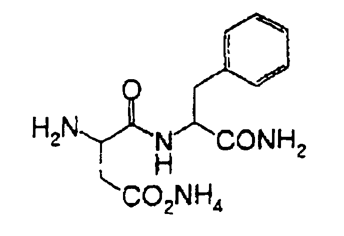 and then the product obtained, optionally released beforehand from its salt, is catalytically hydrogenated and the (α-aspartyl)-β-cyclohexylalaninamide obtained is optionally converted to an addition salt with an acid.

2. Method according to claim 1, **characterized in that** ((L)-α-aspartyl)-(L)-β-cyclohexylalaninamide or its addition salt with an acid is prepared.

3. A method for preparing (α-aspartyl)-phenylalaninamide, its ammonium salt or its addition salt with an acid, **characterized in that** an ester of aspartylphenylalanine of general formula: 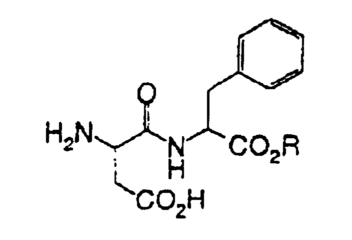 in which R is an alkyl radical comprising 1 to 4 carbon atoms, is amidated, to give the ammonium salt of (α-aspartyl)phenylalaninamide of formula: 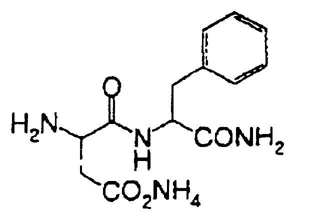 and then the product is optionally released from its salt and is optionally converted to an addition salt with an acid.

4. Method according to claim 3, **characterized in that** ((L)-α-aspartyl)-(L)-phenylalaninamide, its ammonium salt or its addition salt with an acid is prepared.

5. Method according to claim 1 or 3, **characterized in that** the methyl ester of (α-aspartyl)phenylalanine is employed.

6. Method according to claim 1 or 3, **characterized in that** the amidation reaction is carried out by reaction with liquid ammonia.

## Patentansprüche

1. Verfahren zur Herstellung von α-Aspartyl-β-cyclohexylalaninamid, **dadurch gekennzeichnet, daß** man in einer ersten Stufe die Amidierung eines Phenylalanin-aspartyl-esters der allgemeinen Formel 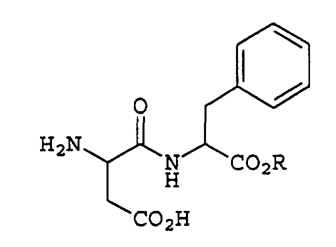 durchführt, in der R ein Rest Alkyl mit 1 bis 4 Kohlenstoffatomen ist, um das Ammoniumsalz des α-Aspartyl-phenylalaninamids der Formel 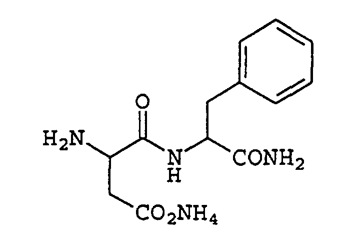 zu ergeben, und man anschließend eine katalytische Hydrierung des erhaltenen, gegebenenfalls aus seinem Salz freigesetzten Produktes durchführt und gegebenenfalls das erhaltene α-Aspartyl-β-cyclohexylalaninamid in ein Additionssalz mit einer Säure überführt.

2. Verfahren nach Anspruch 1, **dadurch gekennzeichnet, daß** man das (L)-α-Aspartyl-(L)-ß-cyclohexylalaninamid oder sein Additionssalz mit einer Säure herstellt.

3. Verfahren zur Herstellung von α-Aspartyl-phenylalaninamid aus seinem Ammoniumsalz oder seinem Additionssalz mit einer Säure, **dadurch gekennzeichnet, daß** man die Amidierung eines Phenylalanin-aspartyl-esters der allgemeinen Formel 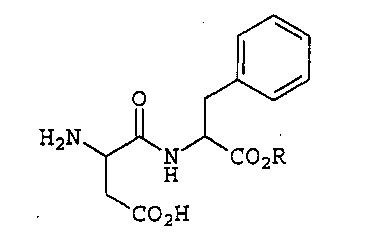 durchführt, in der R ein Rest Alkyl mit 1 bis 4 Kohlenstoffatomen ist, um das Ammoniumsalz des α-Aspartyl-phenylalaninamids der Formel 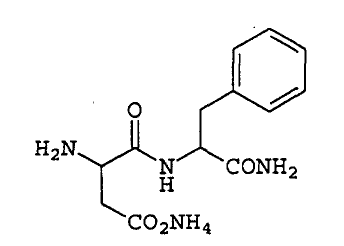 zu ergeben, und man anschließend gegebenenfalls das Produkt aus seinem Salz freisetzt und gegebenenfalls in ein Additionssalz mit einer Säure überführt.

4. Verfahren nach Anspruch 3, **dadurch gekennzeichnet, daß** man das (L)-α-Aspartyl-(L)-phenylalaninamid, sein Ammoniumsalz oder sein Additionssalz mit einer Säure herstellt.

5. Verfahren nach Anspruch 1 oder 3, **dadurch gekennzeichnet, daß** man den Methylester des α-Aspartyl-phenylalanins einsetzt.

6. Verfahren nach Anspruch 1 oder 3, **dadurch gekennzeichnet, daß** man die Reaktion der Amidierung mittels Einwirkung von flüssigem Ammoniak durchführt.
